# EUROPÄISCHE PATENTSCHRIFT

(11) **EP 0 573 459 B1**
(45) Veröffentlichungstag und Bekanntmachung des Hinweises auf die Patenterteilung: **08.05.1996**
(21) Anmeldenummer: 92904671.2
(22) Anmeldetag: 19.02.1992
(51) Int. Cl.: C07C 317/28, C07C 315/02

(54) **VERFAHREN ZUR HERSTELLUNG VON 3'-AMINOPROPYL-2-SULFATOETHYLSULFON**
METHOD FOR PREPARING 3'-AMINOPROPYL-2-SULPHATOETHYLSULPHONE
PROCEDE DE FABRICATION DE LA 3'-AMINOPROPYL-2-SULFATOETHYLSULFONE

(30) Priorität: 27.02.1991 DE 4106106
(43) Veröffentlichungstag der Anmeldung: 15.12.1993
(73) Patentinhaber: HOECHST AKTIENGESELLSCHAFT, D-65926 Frankfurt am Main (DE)
(72) Erfinder: MEIER, Michael, D-6000 Frankfurt am Main 90 (DE); ANGENENDT, Heinrich, D-6000 Frankfurt am Main 71 (DE); GRÖTSCH, Georg, D-6238 Hofheim (DE)
(86) Internationale Anmeldenummer: EP9200349
(87) Internationale Veröffentlichungsnummer: WO9215559

(56) Entgegenhaltungen:
- WO-A-91/13866
- DE-A- 2 040 620
- US-A- 4 450 114

## Beschreibung

Die Erfindung betrifft ein verbessertes Verfahren zur Herstellung von 3'-Aminopropyl-2-sulfatoethylsulfon durch Addition von Mercaptoethanol an Allylamin in wäßriger Schwefelsäure in Gegenwart von Radikalstartern, Oxidation der so erhaltenen Reaktionsmischung mit Wasserstoffperoxid in Anwesenheit katalytischer Mengen Übergangsmetall-Verbindungen und Veresterung mit Schwefelsäure oder Oleum oder Chlorsulfonsäure, in dem man im Reaktionsmedium lösliche Radikalstarter einsetzt.

3'-Aminopropyl-2-sulfatoethylsulfon ist ein wichtiges Vorprodukt zur Herstellung von Reaktivfarbstoffen (EP 0141776).

Die Herstellung von 3'-Aminopropyl-2'-sulfatoethylsulfon wird in der EP 0 518 889 beschrieben, wobei in einem Eintopfverfahren Allylamin mit Mercaptoethanol in wäßriger Schwefelsäure bei Temperaturen von etwa 50 °C bis zum Siedepunkt des Reaktionsgemisches in Gegenwart von Radikalstartern umgesetzt wird, die hierbei anfallende Reaktionsmischung mit Wasserstoffperoxid in Anwesenheit katalytischer Mengen einer Verbindung eines Übergangsmetalls des Periodischen Systems der Elemente bei Temperaturen von etwa 70 bis etwa 100 °C oxidiert wird, mit weiterer Schwefelsäure, bis die Gesamtschwefelsäuremenge im Gemisch mindestens 1 mol, bezogen auf das eingesetzte Allylamin, beträgt, versetzt und das erhaltene 3'-Aminopropyl-2-oxethylsulfon-hemisulfat durch Einengen zur Trockne verestert.

Die Reaktionszeiten für die radikalische Addition von Mercaptoethanol an das Allylamin liegen hierbei zwischen 35 und 45 h.

Es wurde nun überraschenderweise gefunden, daß sich die Reaktionszeit des beschriebenen Verfahrens bei guten Ausbeuten erheblich verkürzen läßt, wenn man im Reaktionsmedium lösliche Radikalstarter, bevorzugt 2,2'-Azobis[2-(2-imidazolin-2-yl)propan] oder dessen Dihydrochlorid oder 2,2'-Azobis(2-amidinopropan) dihydrochlorid, einsetzt und die Umsetzung von Allylamin mit Mercaptoethanol in wäßriger Schwefelsäure bei etwa 25 °C bis zum Siedepunkt des Reaktionsgemisches, bevorzugt bei der Temperatur, bei welcher der Radikalstarter eine Halbwertszeit von etwa 1 bis etwa 5 Stunden hat, durchführt, oxidiert und verestert.

Gegenstand der Erfindung ist somit ein verbessertes Verfahren zur Herstellung von 3'-Aminopropyl-2-sulfatoethylsulfon in guten Ausbeuten und in relativ kurzer Reakionszeit, in dem man in einem Eintopfverfahren Allylamin mit Mercaptoethanol in wäßriger Schwefelsäure bei Temperaturen von etwa 25 °C bis zum Siedepunkt des Reaktionsgemisches in Gegenwart von 2,2'-Azobis[2-(2-imidazolin-2-yl)propan], 2,2'-Azobis[2-(2-imidazolin-2-yl)propan]-dihydrochlorid oder 2,2'-Azobis(2-amidinopropan)dihydrochlorid oder Mischungen daraus umsetzt, die so erhaltene Reaktionsmischung mit Wasserstoffperoxid in Anwesenheit katalytischer Mengen einer Verbindung eines Übergangsmetalls des Periodischen Systems der Elemente als Oxidationskatalysator zum 3'-Aminopropyl-2-oxethylsulfon-hemisulfat oxidiert, mit soviel weiterer Schwefelsäure versetzt, bis die Gesamtschwefelsäuremenge im Gemisch mindestens 1 mol, bezogen auf das eingesetzte Allylamin, beträgt und das nach Oxidation erhaltene 3'-Aminopropyl-2-oxethylsulfon-hemisulfat durch Einengen zur Trockne oder mit Schwefelsäure oder Oleum oder Chlorsulfonsäure in Lösung verestert.

Es ist zweckmäßig, 1 mol Allylamin in wäßriger Schwefelsäure bei einer Temperatur, bei welcher der Radikalstarter eine Halbwertszeit von etwa 1 bis 5 h hat, vorzulegen und 0,9 bis etwa 1,5 mol, vorzugsweise etwa 0,95 bis etwa 1,05 mol Mercaptoethanol, wobei 0,1 bis etwa 5 g, bevorzugt etwa 0,5 bis etwa 1,5 g Radikalstarter pro mol Mercaptoethanol gelöst sind, zuzudosieren. Es ist auch möglich, Mercaptoethanol, wobei 0,1 bis etwa 5 g, bevorzugt etwa 0,5 bis etwa 1,5 g Radikalstarter pro mol Mercaptoethanol gelöst sind, vorzulegen und 1 mol Allylamin in wäßriger Schwefelsäure bei einer Temperatur, bei welcher der Radikalstarter eine Halbwertszeit von etwa 1 bis 5 h hat, zuzudosieren. Man kann jedoch auch so verfahren, daß man 1 mol Allylamin in wäßriger Schwefelsäure und etwa 0,1 bis etwa 1,0 mol Mercaptoethanol bei einer Temperatur, bei welcher der Radikalstarter eine Halbwertszeit von etwa 1 bis 5 h hat, vorzulegen und 0,1 bis 5 g, bevorzugt 0,5 bis 1,5 g Radikalstarter pro mol Mercaptoethanol, im restlichen Mercaptoethanol bzw. in Wasser gelöst, zuzudosieren. Es können auch alle Komponenten gemeinsam vorgelegt werden, jedoch ist dies auf Grund der freiwerdenden Reaktionswärme für eine technische Durchführung kritisch.

Der vorzugsweise Temperaturbereich für 2,2'-Azobis[2-(2-imidazolin-2-yl)propan] oder dessen Dihydrochlorid ist etwa 45 bis ca. 65 °C und für 2,2'-Azobis(2-amidinopropan)dihydrochlorid etwa 55 bis 75 °C.

Als Verbindungen eines Übergangsmetalls des Periodischen Systems der Elemente dienen vorzugsweise Verbindungen des Wolframs oder Vanadins als Oxidationskatalysator, wie beispielsweise Na₂WO₄·2H₂O oder NaVO₃.

Das erfindungsgemäße Verfahren wird zweckmäßigerweise bei Atmosphärendruck durchgeführt; eine Verfahrensdurchführung ist aber auch bei erhöhtem oder vermindertem Druck möglich.

Gegenüber dem in der europäischen Patentanmeldung EP 0 518 889 beschriebenen Verfahrens wird durch die Verwendung von Radikalstartern, die im Reaktionsgemisch löslich sind, wie beispielsweise 2,2'-Azobis[2-(2-imidazolin-2-yl)propan] oder dessen Dihydrochlorid oder 2,2'-Azobis[2-amidinopropan)dihydrochlorid, die Reaktionszeit der Umsetzung von Allylamin und Mercaptoethanol in wäßriger Schwefelsäure auf etwa 3 bis 8 h gegenüber 35 bis 45 h verkürzt. Durch die deutlich verkürzten Reaktionszeiten werden die Raum-Zeit-Ausbeuten stark erhöht, wodurch das Verfahren besonders wirtschaftlich wird.

Durch die nachstehenden Beispiele wird die Erfindung näher erläutert, ohne darauf beschränkt zu werden.

### Beispiel 1

In einem 1-l-Vierhalskolben mit Rührer, Tropftrichter, Thermometer und Rückflußkühler werden 100,0 g Eis und 61,3 g (0,6 mol) Schwefelsäure 96 %ig vorgelegt. Dazu läßt man 57,1 g (1,0 mol) Allylamin zulaufen. Anschließend wird zu diesem Gemisch bei 55 °C 78,1 g (1,0 mol) Mercaptoethanol, das mit 1 g 2,2'-Azobis[2-(2-imidazolin-2-yl)propan]dihydrochlorid versetzt ist, im Verlauf 1 h zudosiert. Dann wird bei 55 bis 60 °C insgesamt 3 h nachgerührt. Nach beendeter Reaktion wird die Lösung mit 0,05 g Natriumwolframat-dihydrat versetzt und innerhalb von 1 h werden 113,3 g (1,0 mol) Wasserstoffperoxid 30 %ig bei 80 °C zudosiert. Um die Temperatur von 80 °C zu halten, muß mit Eis-Wasser gekühlt werden. Dann werden nochmals 113,3 g (1,0 mol) Wasserstoffperoxid 30 %ig bei 80 °C zudosiert. Nach beendeter Dosierung wird 1 h bei 80 °C nachgerührt. Zur Veresterung des angefallenen Oxethylsulfons werden 46,0 g (0,45 mol) Schwefelsäure 96 %ig zugegeben. Bei einer Temperatur von 80 °C/200 mbar wird die Reaktionsmischung in einen Laborkneter getropft. Dann wird die Temperatur langsam auf 150 °C/1 mbar gesteigert und schließlich zur Trockne eingeengt. Es fallen 253,9 g 3'-Aminopropyl-2-sulfatoethylsulfon mit einem Reingehalt von 93,2 % an. Die Ausbeute beträgt 95,4 % d.Th.

### Schmelzpunkt: 235 - 240 °C (Zersetzung)

- ¹H-NMR ([D₆]DMSO):: δ = 2,0 (q, J=7Hz; CH₂CH₂CH₂CH₂, 2H), 2,9 (m; CH₂NH₃ ⁺; 2H), 3,2 (m; SO₂CH₂CH₂; 2H), 3,4 (t, J=7Hz; CH₂CH₂SO₂), 4,1 (t, J=7Hz; CH₂OSO⁻; 2H), 7,7 (breit; NH₃ ⁺; 3H).
- IR (KBr):: 3160, 2990, 2935, 1320, 1290, 1205, 1060 cm⁻¹.

### Beispiel 2

In einem 1-l-Vierhalskolben mit Rührer, Tropftrichter, Thermometer und Rückflußkühler werden 100,0 g Eis und 61,3 g (0,6 mol) Schwefelsäure 96 %ig vorgelegt. Man läßt nun 57,1 g (1,0 mol) Allylamin zulaufen. Anschließend werden 78,1 g (1,0 mol) Mercaptoethanol, die mit 1 g 2,2'-Azobis(2-amidinopropan)dihydrochlorid versetzt sind, bei ca. 70 °C zudosiert. Anschließend wird 4 h bei dieser Temperatur nachgerührt. Nach beendeter Reaktion wird bei 80 °C mit 0,025 g Natriumwolframatdihydrat versetzt und innerhalb von 1 h 97,1 g (1,0 mol) Wasserstoffperoxid 35 %ig zudosiert. Um die Temperatur von 80 °C zu halten, wird mit Eis-Wasser gekühlt. Dann werden nochmals 97,1 g (1,0 mol) Wasserstoffperoxid 30 %ig bei 80 °C zudosiert. Nach beendeter Zudosierung wird 1 h bei 80 °C nachgerührt. Zur Veresterung des Oxethylsulfons werden 46,0 g (0,45 mol) Schwefelsäure 96 %ig zugegeben. Bei einer Temperatur von 80 °C/200 mbar wird das Reaktionsgemisch in einen Laborkneter getropft und langsam die Temperatur auf 150 °C/1 mbar gesteigert und zur Trockne eingeengt. Es fallen 251,5 g 3'- Aminopropyl-2-sulfatoethylsulfon an mit einem Reingehalt von 95,3 %. Die Ausbeute beträgt somit 96,9 % d.Th..

### Schmelzpunkt: 235 - 240 °C (Zersetzung)

Die spektroskopischen Daten sind mit denen, die in Beispiel 1 angegeben sind, identisch.

### Beispiel 3

In einem 1-l-Vierhalskolben mit Rührer, Tropftrichter, Thermometer und Rückflußkühler werden 100,0 g Eis und 61,3 g (0,6 mol) Schwefelsäure 96 %ig vorgelegt. Man läßt nun 57,1 g (1,0 mol) Allylamin zulaufen. Zu diesem Gemisch werden 39,05 g (0,5 mol) Mercaptoethanol zudosiert. Dann wird auf 50 °C geheizt. Über einen Zeitraum von 30 min werden zu diesem Reaktionsgemisch 39,05 g (0,5 mol) Mercaptoethanol, das mit 1 g 2,2'-Azobis[2-(2-imidazolin-2-yl)propan]dihydrochlorid versetzt ist, zudosiert. Dann wird bei 60 °C insgesamt 3 h nachgerührt. Die weitere Umsetzung erfolgt, wie in Beispiel 1 beschrieben. Es fallen 250,3 g 3'-Aminopropyl-2-sulfatoethylsulfon mit einem Reingehalt von 93,8 % an. Die Ausbeute beträgt somit 94,9 % d.Th..

### Schmelzpunkt: 235 - 240 °C (Zersetzung)

Die spektroskopischen Daten sind mit denen, die in Beispiel 1 angegeben sind, identisch.

### Beispiel 4

In einem 1-l-Vierhalskolben mit Rührer, Tropftrichter, Thermometer und Rückflußkühler werden 100,0 g Eis und 61,3 g (0,6 mol) Schwefelsäure 96 %ig vorgelegt. Dazu läßt man 57,1 g (1,0 mol) Allylamin zulaufen. Anschließend werden zu diesem Gemisch bei 55 °C 78,1 g (1,0 mol) Mercaptoethanol, das mit 1 g 2,2'-Azobis[2-(2-imidazolin-2-yl)propan]dihydrochlorid versetzt ist, im Verlauf 1 h zudosiert. Dann wird bei 55 bis 60 °C insgesamt 3 h nachgerührt. Nach beendeter Reaktion wird die Lösung mit 0,05 g Natriumwolframat-dihydrat versetzt und innerhalb von 1 h werden 113,3 g (1,0 mol) Wasserstoffperoxid 30 %ig bei 80 °C zudosiert. Um die Temperatur von 80 °C zu halten, muß mit Eis-Wasser gekühlt werden. Dann werden nochmals 113,3 g (1,0 mol) Wasserstoffperoxid 30 %ig bei 80 °C zudosiert. Nach beendeter Dosierung wird 1 h bei 80 °C nachgerührt.

Die Oxidationslösung wird im Vakuum eingedampft, bis sie eben noch rührbar ist. Anschließend werden 47,6 g 100 %ige Schwefelsäure und 199,4 g 65 %iges Oleum zugegeben. Nach 2 h Nachrühren bis 120 °C wird auf 25 °C gekühlt und das Gemisch in kaltes Ethanol/Wasser-Gemisch eingetragen. Das entstandene Kristallisat wird abgesaugt, gut gewaschen und getrocknet. Die Ausbeute beträgt 235,9 g (95,4 % d.Th.) an 3'-Aminopropyl-2-sulfatoethylsulfon (Gehalt: 95 Gew.-%).

### Schmelzpunkt: 235-240 °C (Zersetzung)

Die spektroskopischen Daten sind mit denen, die in Beispiel 1 angegeben sind, identisch.

### Beispiel 5

In einem 1-l-Vierhalskolben mit Rührer, Tropftrichter, Thermometer und Rückflußkühler werden 100,0 g Eis und 61,3 g (0,6 mol) Schwefelsäure 96 %ig vorgelegt. Dazu läßt man 57,1 g (1,0 mol) Allylamin zulaufen. Anschließend werden zu diesem Gemisch bei 55 °C 78,1 g (1,0 mol) Mercaptoethanol, das mit 1 g 2,2'-Azobis[2-(2-imidazolin-2-yl)propan]dihydrochlorid versetzt ist, im Verlauf 1 h zudosiert. Dann wird bei 55 bis 60 °C insgesamt 3 h nachgerührt. Nach beendeter Reaktion wird die Lösung mit 0,05 g Natriumwolframat-dihydrat versetzt und innerhalb von 1 h werden 113,3 g (1,0 mol) Wasserstoffperoxid 30 %ig bei 80 °C zudosiert. Um die Temperatur von 80 °C zu halten, muß mit Eis-Wasser gekühlt werden. Dann werden nochmals 113,3 g (1,0 mol) Wasserstoffperoxid 30 %ig bei 80 °C zudosiert. Nach beendeter Dosierung wird 1 h bei 80 °C nachgerührt.

Die Oxidationslösung wird im Vakuum eingedampft, bis der Ansatz gerade noch rührbar ist. Anschließend werden 169,5 g 100 %ige Schwefelsäure und 133,7 g Chlorsulfonsäure bei 80 °C zugegeben. Danach wird auf 25 °C gekühlt und das Gemisch in eiskaltes Ethanol/Wasser-Gemisch eingetragen. Das entstandene Kristallisat wird abgesaugt und gewaschen. Nach dem Trocknen erhält man 225,4 g (91,1 % d.Th.) an 3'-Aminopropyl-2-sulfatoethylsulfon (Gehalt: 95 Gew.-%).

### Schmelzpunkt: 235-240 °C (Zersetzung)

Die spektroskopischen Daten sind mit denen, die in Beispiel 1 angegeben sind, identisch.

### Beispiel 6

In einem 1-l-Vierhalskolben mit Rührer, Tropftrichter, Thermometer und Rückflußkühler werden 100,0 g Eis und 61,3 g (0,6 mol) Schwefelsäure 96 %ig vorgelegt. Dazu läßt man 57,1 g (1,0 mol) Allylamin zulaufen. Anschließend werden zu diesem Gemisch bei 55 °C 78,1 g (1,0 mol) Mercaptoethanol, das mit 1 g 2,2'-Azobis[2-(2-imidazolin-2-yl)propan] versetzt ist, im Verlauf 1 h zudosiert. Dann wird bei 55 bis 60 °C insgesamt 3 h nachgerührt. Nach beendeter Reaktion wird die Lösung mit 0,05 g Natriumwolframat-dihydrat versetzt und innerhalb von 1 h werden 226,6 g (2,0 mol) Wasserstoffperoxid 30 %ig bei 100 °C zudosiert. Nach beendeter Dosierung wird 1 h bei 100 °C nachgerührt.

Die Oxidationslösung wird im Vakuum eingedampft, bis der Ansatz gerade noch rührbar ist. Anschließend werden 169,5 g 100 %ige Schwefelsäure und 133,7 g Chlorsulfonsäure bei 80 °C zugegeben. Danach wird auf 25 °C gekühlt und das Gemisch in eiskaltes Ethanol/Wasser-Gemisch eingetragen. Das entstandene Kristallisat wird abgesaugt und gewaschen. Nach dem Trocknen erhält man 225,3 g (91,1 % d.Th.) an 3'-Aminopropyl-2-sulfatoethylsulfon (Gehalt: 96,8 Gew.-%).

### Schmelzpunkt: 235-240 °C (Zersetzung)

Die spektroskopischen Daten sind mit denen, die in Beispiel 1 angegeben sind, identisch.

## Patentansprüche

1. Verfahren zur Herstellung von 3'-Aminopropyl-2-sulfatoethylsulfson in guter Ausbeute, dadurch gekennzeichnet, daß man in einem Eintopfverfahren Allylamin mit Mercaptoethanol in wäßriger Schwefelsäure bei Temperaturen von etwa 25°C bis zum Siedepunkt des Reaktionsgemisches in Gegenwart von 2,2'-Azobis[2-(2-imidazolin-2-yl)propan], 2,2'-Azobis[2-(2-imidazolin-2-yl)propan]-dihydrochlorid oder 2,2'-Azobis(2-amidinopropan)dihydrochlorid oder Mischungen daraus umsetzt, die so erhaltene Reaktionsmischung mit Wasserstoffperoxid in Anwesenheit katalytischer Mengen einer Verbindung eines Übergangsmetalls des Periodischen Systems der Elemente als Oxidationskatalysator zum 3'-Aminopropyl-2-oxethylsulfon-hemisulfat oxidiert, mit soviel weiterer Schwefelsäure versetzt, bis die Gesamtschwefelsäuremenge im Gemisch mindestens 1 mol, bezogen auf das eingesetzte Allylamin, beträgt und das nach Oxidation erhaltene 3'-Aminopropyl-2-oxethylsulfon-hemisulfat durch Einengen zur Trockne oder mit Schwefelsäure oder Oleum oder Chlorsulfonsäure in Lösung verestert.

2. Verfahren nach Anspruch 1, dadurch gekennzeichnet, daß die Umsetzung von Allylamin mit Mercaptoethanol in wäßriger Schwefelsäure bei der Temperatur, bei welcher der Radikalstarter eine Halbwertszeit von etwa 1 bis etwa 5 h hat, durchgeführt wird.

3. Verfahren nach mindestens einem der Ansprüche 1 und 2, dadurch gekennzeichnet, daß bei Verwendung von 2,2'-Azobis[2-(2-imidazolin-2-yl)propan] oder dessen Dihydochlorid die Umsetzung von Allylamin mit Mercaptoethanol in wäßriger Schwefelsäure bei etwa 45° bis 65°C duchgeführt wird.

4. Verfahren nach mindestens einem der Anspüche 1 und 2, dadurch gekennzeichnet, daß bei Verwendung von 2,2'-Azobis(2-amidinopropan)dihydrochlorid die Umsetzung von Allylamin in wäßriger Schwefelsäure mit Mercaptoethanol bei etwa 55° bis etwa 75°C durchgeführt wird.

5. Verfahren nach mindestens einem der Anspüche 1 bis 4, dadurch gekennzeichnet, daß in Gegenwart einer Verbindung des Wolframs oder Vanadiums als Oxidationskatalysator oxidiert wird.

6. Verfahren nach mindestens einem der Ansprüche 1 bis 5, dadurch gekennzeichnet, daß in Gegenwart von Na₂WO₄·2H₂O als Oxidationskatalysator oxidiert wird.

7. Verfahren nach mindestens einem der Ansprüche 1 bis 5, dadurch gekennzeichnet, daß in Gegenwart von NaVO₃ als Oxidationskatalysator oxidiert wird.

8. Verfahren nach mindestens einem der Anspüche 1 bis 7, dadurch gekennzeichnet, daß die Reaktion bei vermindertem Druck, bei Atmosphärendruck oder bei Überdruck durchgeführt wird.

## Claims

1. A process for the preparation of 3'-aminopropyl 2-sulfatoethyl sulfone in high yield, which comprises reacting allylamine with mercaptoethanol in aqueous sulfuric acid in a one-pot process at temperatures from about 25°C to the boiling point of the reaction mixture in the presence of 2,2'-azobis[2-(2-imidazolin-2-yl)-propane], 2,2'-azobis[2-(2-imidazolin-2-yl)propane] dihydrochloride or 2,2'-azobis(2-amidinopropane) dihydrochloride or mixtures thereof, oxidizing the reaction mixture thus obtained with hydrogen peroxide in the presence of catalytic amounts of a compound of a transition metal of the periodic table of elements as oxidation catalyst to give 3'-aminopropyl 2-hydroxyethyl sulfone hemisulfate, adding further sulfuric acid until the total amount of sulfuric acid in the mixture is at least 1 mol, relative to the allylamine used, and esterifying the 3'-aminopropyl 2-hydroxyethyl sulfone hemisulfate obtained after oxidation by evaporation to dryness or with sulfuric acid or oleum or chlorosulfonic acid in solution.

2. The process as claimed in claim 1, wherein the reaction of allylamine with mercaptoethanol is carried out in aqueous sulfuric acid at the temperature at which the free-radical initiator has a half-life of about 1 to about 5 hours.

3. The process as claimed in at least one of claims 1 and 2, wherein when 2,2'-azobis[2-(2-imidazolin-2-yl)-propane] or its dihydrochloride is used, the reaction of allylamine with mercaptoethanol is carried out in aqueous sulfuric acid at about 45° to 65°C.

4. The process as claimed in at least one of claims 1 and 2, wherein when 2,2'-azobis(2-amidinopropane) dihydrochloride is used, the reaction of allylamine in aqueous sulfuric acid with mercaptoethanol is carried out at about 55° to about 75°C.

5. The process as claimed in at least one of claims 1 to 4, wherein the oxidation is carried out in the presence of a compound of tungsten or vanadium as oxidation catalyst.

6. The process as claimed in at least one of claims 1 to 5, wherein the oxidation is carried out in the presence of Na₂WO₄×2H₂O as oxidation catalyst.

7. The process as claimed in at least one of claims 1 to 5, wherein the oxidation is carried out in the presence of NaVO₃ as oxidation catalyst.

8. The process as claimed in at least one of claims 1 to 7, wherein the reaction is carried out at reduced pressure, at atmospheric pressure or at superatmospheric pressure.

## Revendications

1. Procédé pour la préparation de la 3'-aminopropyl-2-sulfatoéthylsulfone avec de bons rendements, caractérisé en ce que l'on fait réagir dans un procédé à un réacteur l'allylamine avec le mercaptoéthanol en milieu d'acide sulfurique aqueux à des températures d'environ 25 °C jusqu'au point d'ébullition du mélange réactionnel, en présence de 2,2'-azobis[2-(2-imidazolin-2-yl)-propane], de dichlorhydrate de 2,2'-azobis-[2-(2-imidazolin-2-yl]-propane ou de dichlorhydrate de 2,2'-azobis-(2-amidinopropane) ou de leurs mélanges, on oxyde le mélange réactionnel obtenu avec le peroxyde d'hydrogène, en présence de quantités catalytiques d'un composé de métal de transition du Tableau Périodique des Eléments en tant que catalyseurs d'oxydation pour obtenir l'hémisulfate de 3'-aminopropyl-2-oxéthylsulfone, on y ajoute une telle quantité supplémentaire d'acide sulfurique à ce que la quantité totale en acide sulfurique dans le mélange soit d'au moins 1 mole par rapport à l'allylamine utilisée, et on estérifie en solution l'hémisulfate de 3'-aminopropyl-2-oxéthylsulfone obtenu par évaporation jusqu'à siccité ou par l'acide sulfurique ou par l'oléum ou par l'acide chlorosulfonique.

2. Procédé selon la revendication 1, caractérisé en ce que l'on met en oeuvre la réaction de l'allylamine avec le mercaptoéthanol en milieu d'acide sulfurique aqueux à la température à laquelle la demi-vie de l'amorceur radicalaire est d'environ 1 à environ 5 heures.

3. Procédé selon au moins l'une des revendications 1 et 2, caractérisé en ce que l'on met en oeuvre la réaction de l'allylamine avec le mercaptoéthanol en milieu d'acide sulfurique aqueux à une température d'environ 45 à 65 °C en utilisant le 2,2'-azobis-[2-(2-imidazolin-2-yl)-propane] ou son dichlorhydrate.

4. Procédé selon au moins l'une des revendications 1 et 2, caractérisé en ce que l'on met en oeuvre la réaction de l'allylamine en milieu d'acide sulfurique concentré avec le mercaptoéthanol à environ 55 à environ 75 °C en utilisant le dichlorhydrate de 2,2'-azobis-(2-amidinopropane).

5. Procédé selon au moins l'une des revendications 1 et 4, caractérisé en ce que l'on oxyde en présence d'un composé de tungstène ou de vanadium en tant que catalyseur d'oxydation.

6. Procédé selon au moins l'une des revendications 1 et 5, caractérisé en ce que l'on oxyde en présence de Na₂WO₄·2H₂O en tant que catalyseur d'oxydation.

7. Procédé selon au moins l'une des revendications 1 et 5, caractérisé en ce que l'on oxyde en présence de NaVO₃ en tant que catalyseur d'oxydation.

8. Procédé selon au moins l'une des revendications 1 et 7, caractérisé en ce que l'on met en oeuvre la réaction sous pression réduite, à la pression atmosphérique ou sous pression élevée.
